# EUROPEAN PATENT APPLICATION

(11) **EP 3 327 119 A1**
(43) Date of publication of application: **30.05.2018**
(21) Application number: 17210574.4
(22) Date of filing: 26.11.2012
(51) Int. Cl.: C12N 7/02, C12N 15/86

(54) **SCALABLE LENTIVIRAL VECTOR PRODUCTION SYSTEM COMPATIBLE WITH INDUSTRIAL PHARMACEUTICAL APPLICATIONS**

(30) Priority: 24.11.2011 EP 11306551; 24.11.2011 US 201161563566 P
(62) Divisional of application: 12795778.5
(71) Applicant: Genethon, 91000 Evry (FR)
(72) Inventor: MARCEAU, Nicolas, 91600 Savigny sur Orge (FR); GASMI, Mehdi, Sarasota, FL 34243 (US)
(74) Representative: Cabinet Becker et Associés

(57) **Abstract**

The present invention relates to the industrialization of the production of recombinant lentiviral vectors in order to manufacture sufficient materials for therapeutic applications such as gene therapy and/or DNA vaccination, for use in clinical trials and/or commercial use.

## Description

The present invention relates to the industrialization of the production of recombinant lentiviral vectors in order to manufacture sufficient materials for therapeutic applications such as gene therapy and/or DNA vaccination, for use in clinical trials and/or commercial use.

### BACKGROUND OF THE INVENTION

Advances in the use of recombinant viral vectors for gene therapy and DNA vaccination applications have created a need for large-scale manufacture of clinical-grade viral vectors for transfer of genetic materials. One such family of viral vectors is the genus of lentiviruses within the retrovirus family of viruses.

Lentiviral vectors used in gene therapy applications are conventionally manufactured by calcium phosphate transfection of adherent cells which require fetal bovine serum in the culture media, with a lentiviral construct DNA system (Merten et al., 2011, Hum Gene Ther. 22(3):343-56). The presence of this animal derived component in the culture constitutes a safety risk that limits the GMP compliance of the method. In addition this method of production is severely limited in terms of scale up and is not adapted to the production of large amounts of vector particles required for therapeutic, commercial and/or industrial applications of gene therapy. For example, the current conventional method allows the generation in one run, and before purification, of 24 to 48 L of lentiviral vector suspension at a titer of approximately 1x10⁷ to 3x10⁷ functional vector particles per mL which, with a standard purification yield of 20%, would generate at best 3x10¹¹ particles in the final product. In comparison, a phase I clinical trial would require at least 5 x 10¹¹ functional lentiviral vector particles (McGregor et al., 2001, Hum Gene Ther., 12:2028-2029). Therefore there is a strong need today for industrial lentiviral vector biomanufacturing processes that would accommodate the need for sufficient quantities of therapeutic vectors when either a large amount of vector is required per patient or when large numbers of patients must be treated or more generally for keeping costs at reasonable levels and maintaining good manufacturing practice (GMP) compliance.

One potential avenue for improvement is the use of cell cultures in suspension growing in chemically conditioned media in the absence of fetal bovine serum (FBS) to overcome the safety risk linked to the use of FBS but also the need for cell culture vessels that are the major culprit of the lack of scalability of the conventional process. For example, the production of viral vectors by transient transfection in suspension cultures in the absence of serum has recently been described. In particular, Ansorge et al. have proposed a process for the production of lentiviruses by transient transfection of suspension-grown HEK293SF-3F6 cells in perfusion cultures (Ansorge et al., 2009, J Gene Med, 11: 868-876). However, the method proposed is both complicated and limited in scale. Indeed, the method of Ansorge et al. is performed in perfusion cultures which necessitate several harvesting steps and complicated control measures. In addition, the production proposed in that study is limited to a volume of 3 liters. Segura et al. have also proposed a process for the production of lentiviral vectors by transient transfection of suspension cultures (Segura et al., 2007, Biotechnology and Bioengineering, 98 (4): 789-799). Nonetheless, the method proposed is complicated because it requires several harvesting steps with total media replacement at days 3, 4 and 5 post-transfection and complex control measures. In addition, viral vector production is limited to a volume of 3 liters and only recombinant protein production, but not viral vector production, is reported to have been scaled up to 60 L-scale. Accordingly, despite these reports, there remains a need for the development of a straightforward industrial process for lentiviral vector production from suspension cell culture which addresses both quantitative and qualitative issues that are imposed upon a commercial-scale lentiviral-based vaccine and/or gene therapy product. The present invention addresses and meets these needs by disclosing an optimized cell culture and lentivirus production process, resulting in improved virus productivity.

### SUMMARY OF THE INVENTION

The present invention relates to a method for the industrial scale production of pharmaceutical grade recombinant lentiviral vectors. The results presented below show that the inventors have been able to provide a method that is as good as or better in terms of productivity and quality than existing GMP production methods using adherent cells, but which has a much more scalable production potential.

Thus, in a first aspect, the invention relates to a method for the production of a recombinant lentiviral vector, lentivirus and pseudovirus, comprising:
- culturing, in suspension in a serum-free medium, mammalian cells transfected with at least one plasmid adapted for the production of a lentiviral vector, the culture being carried out in a volume of at least 5 L;
- harvesting the produced recombinant lentiviral vector from the culture medium.

In a preferred embodiment, the mammalian cells are Human Embryonic Kidney 293T cells (also referred to as HEK293T cells or 293T cells) capable of growing in suspension under serum-free conditions. These cells have been shown by the inventors to be particularly suited for the industrialization of the production of large amounts of recombinant lentiviral vector meeting both quantitative and qualitative requirements for use in therapy, in particular in gene therapy clinical trials and commercial applications.

In a particular embodiment, the lentiviral vectors are harvested between 36 hours and 72 hours post-transfection, preferably after 48 hours. In a further embodiment, the culture is implemented in at least a 10 L scale, or preferably of at least 50 L scale, or even preferably of at least100 L and can be particularly adapted to an industrial production of lentiviral vectors allowing harvesting of at least 10⁷ infectious genomes IG/mL. The method of the invention is the first ever that allows industrial lentivirus production, and very high levels of viral vectors will be achieved as is shown by the linearity of the scale-up from 100 mL to 50 L presented in the experimental part. Therefore, very high levels of viral vectors will be achievable by implementing this method (for example, at a scale of 1000 L). In another preferred embodiment, the harvesting step consists of a single lentivirus harvest. To the inventors' knowledge, this is the first report of the production of lentiviral vectors at such a high scale implementing a single harvest. This embodiment has the advantage of providing a straightforward method requiring as few steps as possible and allowing the control of costs.

In addition, the present invention also relates to the above method wherein the transfection of the mammalian cells is a transient transfection and the harvesting step consists of a single harvest implemented between 48 and 72 hours post-transfection.

The invention further discloses optimizations in the transfection process before culturing the cells for lentivirus production. In a particular embodiment, the cells are transfected with a mixture of polyethylenimine (PEI) and plasmids. In a specific embodiment, the above method comprises a transfection step wherein the cells are transfected with such a mixture of PEI and plasmids. In a particular variant, the transfection is carried out with a total plasmid DNA amount of at least 1.5 µg/10⁶ cells. In another specific variant, the PEI is a 20 - 25 kD linear PEI. In a further specific variant, an optimization provided with the present invention also relates to the relative amounts of each component of the mixture. In particular, in a specific variant of the invention the PEI and the plasmids are mixed before transfection according to a N/P ratio of less than 10, wherein N/P refers to the number of nitrogen atoms in the PEI per oligonuclotide phosphate. In a preferred variant, the N/P ratio is around 6. In a further specific variant, the contact time between the PEI and the plasmids before addition to the cell culture has also been explored and may ideally be comprised between 5 and 30 minutes, the contact time being in particular of around 10 minutes.

The method for production of the invention can advantageously be optimized by adding sodium butyrate to the cell culture 24 hours post-transfection, without changing the medium. Preferably, sodium butyrate is added to the culture at a final concentration comprised between 2 mM and 12 mM, in particular between 2 mM and 10 mM or between 5 mM and 12 mM (for example around 5, 8 or 12 mM), more particularly at a final concentration of 5 mM.

In a further embodiment of the method of the invention, the plasmids transfected in the cells comprise four plasmids, including a plasmid encoding envelope proteins (Env plasmid), which may be derived from the lentivirus in question, but may also be derived from other enveloped viruses, a plasmid encoding lentiviral Gag and Pol proteins (Gag-Pol plasmid), a plasmid encoding a lentiviral Rev protein (Rev plasmid) and a plasmid comprising a transgene of interest (TOI) expression cassette between a lentiviral 3'-LTR and a lentiviral 5'LTR (TOI plasmid).

In a further aspect, the invention provides a cell culture device (or bioreactor), wherein said culture device contains a volume of at least 5 L of a serum-free culture medium comprising mammalian cells transfected with at least one plasmid adapted for the production of a lentiviral vector, said cells growing in suspension in said culture device. In a particular embodiment, the cells in the serum-free culture medium are HEK 293T cells.

In another aspect, the invention relates to a method for optimizing the production of a lentiviral vector by a mammalian cell grown in suspension in a serum-free medium, transfected with plasmids required for said production, comprising adding sodium butyrate 24 hours post-transfection to the culture without changing the medium of the culture. Preferably, sodium butyrate is added at a final concentration of 5 mM.

### DETAILED DESCRIPTION OF THE INVENTION

The present invention relates to a method for the industrial scale production of pharmaceutical grade recombinant lentiviral vectors. Produced vectors may be useful for the treatment of conditions in an animal subject, in particular a mammal, more particularly in a human subject in need thereof.

### Plasmids for the production of lentiviruses

Lentiviruses are exogenous retroviruses of mammals and form one genus of the retroviridae. Lentiviral vectors (LV) are derived from a number of primate lentiviruses such as human immunodeficiency virus (HIV)-1 or -2, or various simian immunodeficiency viruses (SIV), or from nonprimate lentiviruses such as equine infectious anemia virus (EIAV), feline immunodeficiency virus (FIV), or caprine arthritis-encephalitis virus (CAEV).

The lentiviral components useful for the production of a recombinant lentiviral vector are known in the art. See for example Zufferey et al., 1997, Nat. Biotechnol. 15:871-875 and Dull et al., 1998, J. Virol. 72(11):8463-8471. A "second generation" lentiviral vector system refers to a packaging system that lacks functional accessory genes, such as one from which the accessory genes vif, vpr, vpu and nef, have been deleted or inactivated (Zufferey et al., cited *supra*). A "third generation" lentiviral vector system refers to a lentiviral packaging system that has the characteristics of a second generation vector system, and further lacks a functional tat gene, such as one from which the tat gene has been deleted or inactivated. Typically, the gene encoding Rev is provided on a separate expression construct (See, e.g., Dull et al., cited *supra*). For a more recent summary of lentiviral vector systems that can be used for production of a recombinant lenviral vector, see also Schweizer and Merten, 2010, Current Gene Therapy 10(6), 474-486, most particularly part 2.2 ("lentiviral vector systems"). Schweizer and Merten report non industrialisable processes.

The different functions necessary for the production of a lentiviral vector can be provided to the cells by any number of plasmids. In particular, these functions may be provided by at least one, two, three or four plasmids. In a particular embodiment of the invention, the different functions necessary for production of a lentiviral vector are provided to the mammalian cell (in particular a 293T cell growing in suspension under serum-free conditions) by the transfection, in particular transient transfection, of four plasmids adapted for producing lentiviral vectors, wherein one plasmid encodes envelope proteins (Env plasmid), one plasmid encodes lentiviral Gag and Pol proteins (Gag-Pol plasmid), one plasmid encodes a lentiviral Rev protein (Rev plasmid) and one plasmid comprises a transgene of interest (TOI) expression cassette between a lentiviral 3'-LTR and a lentiviral 5'LTR (TOI plasmid).

Each function (or component) can be derived from any suitable lentivirus. However, in a preferred embodiment, the *gag-pol, rev* and the lentiviral genome (3'-LTR and a 5'LTR) are derived from a HIV virus, in particular from HIV-1 or HIV-2.

In addition, the recombinant lentiviral vector can be a pseudotyped vector, comprising a modified envelope protein, an envelope protein derived from a different virus or a chimeric envelope protein. Accordingly, for example, the Env plasmid can encode a VSV-G Env protein, although those skilled in the art will appreciate that other env genes may be employed.

Of course, the TOI used in the plasmid(s) will depend on the specific use intended for the lentiviral vector. Illustrative, non limiting, examples of TOI include a TOI encoding a therapeutic RNA (e.g. a TOI encoding an antisense RNA complementary to a target RNA or DNA sequence), a gene therapy TOI encoding a protein defective or absent in a diseased subject, and a vaccine TOI used for DNA vaccination, i.e. encoding a protein the expression of which will induce vaccination of the recipient organism against said protein.

### Mammalian suspension cells

Mammalian cells for the production of lentiviruses are known in the art. Representative examples of such cells include Human Embryonic Kidney (HEK) 293 cells and derivatives thereof (for example the 293SF-3F6 line) selected for their ability to grow in suspension under serum-free conditions and which are ideally highly transfectable. Other cell types include, but are not limited to, HeLa cells, A549 cells, KB cells, CKT1 cells, NIH/sT3 cells, Vero cells, Chinese Hamster Ovary (CHO) cells, or any eukaryotic cell which support the lentivirus life cycle.

In a particular embodiment, the cells are 293T cells, which are well known in the art. 293T are commercially available from a number of suppliers. These cells correspond to a cell line derived from human embryonic kidney cells transformed with SV40 large-T antigen requiring fetal bovine serum for growth. Specifically, the HEK 293 cell line originally was derived from human embryonic kidney cells transfected with fragments of mechanically sheared human adenovirus type 5 (Ad5) through selection of cells that showed many of the characteristics of Ad transformation. The transforming region of human adenovirus contains early region 1 (E1), consisting of two transcription units, E1a and E1b, which products are necessary and sufficient for mammalian cell transformation by Ads. These 293 cells are a subclone of the original Frank Graham 293 cells which were selected for higher virus yield (probably adenovirus) and better cell growth (Graham et al, 1977, J Gen Virol, 36, 59-74). From HEK 293 cells, the 293T cell line was created in the laboratory of Michele Calos (Stanford University) by transfection with a gene encoding the SV-40 T-antigen and a neomycin resistance gene.
Adherent 293T cells have been previously used for producing lentiviral vectors. However, the present inventors are the first to propose an efficient method for producing lentiviral vectors from these cells adapted to culture conditions in suspension in the absence of serum to accommodate for industrial scale production of lentiviral vectors. Indeed, in the examples of this application, the inventors present for the first time a method for producing lentiviral vectors the scale-up of which is linear from 100 mL to 50 L. Therefore, very high levels of viral vectors will be achievable by implementing this method (for example, at a scale of 1000 L).

The cells are cultured in a serum-free medium selected with respect to the specific cell used and permitting the production of the lentiviral vector. The serum-free medium allows production of lentiviral vector suitable for therapeutic applications.. For a review on serum-free media, see Chapter 9 (serum-free media) of Culture of Animal Cells: A Manual of Basic Technique; Ed. Freshen, RI, 2000, Wiley-Lisps, pp. 89-104 and 105-120. In general, serum free media will be manipulated to enhance growth of the respective cell line in culture, with a potential for inclusion of any of the following: a selection of secreted cellular proteins, diffusible nutrients, amino acids, organic and/or inorganic salts, vitamins, trace metals, sugars, and lipids as well as perhaps other compounds such as growth promoting substances (e.g., cytokines). It is also desirable that such media are supplemented with glutamine or an alternative to glutamine such as GlutaMAX™, as disclosed herein. Such media are commercially available, and with the further knowledge of the present invention the person skilled in the art will be able to select the appropriate ones with respect to the mammalian host cells. The medium may be supplemented with additives such as a non-ionic surfactant such as Pluronic® F68 (Invitrogen, catalogue No. 24040-032), used for controlling shear forces in suspension cultures, an anti-clumping agent (e.g. from Invitrogen, catalogue No. 0010057AE) and L-glutamine or an alternative to L-glutamine such as a L-alanyl-L-glutamine dipeptide, e.g. GlutaMAX™ (Invitrogen, catalogue No 35050-038). The media and additives used in the present invention are advantageously GMP compliant. For example, representative commercially available serum-free media which can be used for growing 293T cells in suspension include F17 medium® (Invitrogen) and Ex-Cell 293® (SAFC). In particular, 293T cells can be grown in customized F17 medium® supplemented with additives preventing the formation of cell aggregates. In particular, the method of the present invention is herein shown to be improved when F17 medium® is supplemented with Pluronic® F68 between 0,05% and 0,1% and more particularly at 0,08%, , GIBCO® Anti-Clumping Agent between 0,01% and 0,1% and more particularly 0,01% and GlutaMAX™ between 2 and 6mM and more particularly at 4 mM final concentration. These additives used in the amounts herein provided are advantageous in that they allow to optimally prevent 293T cell aggregates.

Advantageously, the media and additives used in the present invention being serum-free and animal component free, they respect GMPs and thus allow industrial production of lentiviral vectors for use in animal, in particular human, therapy.

In a particular embodiment, the cells can be used at a cell density comprised between 0.8 and 1.3x10⁶ cells/mL.

### Transient transfection

In the method of the present invention mammalian cells, in particular 293T cells as described above are transfected with one or more plasmid(s) adapted for the production of a lentiviral vector. Preferably, the transfection is a transient transfection.

Various techniques known in the art may be employed for introducing nucleic acid molecules into cells. Such techniques include chemical-facilitated transfection using compounds such as calcium phosphate, cationic lipids, cationic polymers, liposome-mediated transfection, non-chemical methods such as electroporation, particle bombardment, or microinjection, and infection with a virus that contains the nucleic acid molecule of interest (sometimes termed "transduction").

However, according to a preferred embodiment of the invention, transient transfection is carried out using polyethylenemine (PEI) as a transfection reagent. PEI has high gene transfer activity in many cell lines while displaying low cytotoxicity, is cost-effective and therefore is compatible with industrial scale production applications. This polymer is available as both linear and branched isomers with a wide range of molecular weights and polydispersities, which physicochemical parameters are critical for efficient gene transfer activity (Godbey W. T. et al., J. Control Release, 60,149160 (1999). In a particular embodiment, the PEI used in the present invention is a 20 - 25 kD linear PEI. For example, in a particular embodiment, the PEI used in the present invention is JetPEI® or PEIPro® (both available from PolyPlus). JetPEI® and PEIPro® transfection reagents are linear polyethylenimine derivatives, free of components of animal origin, providing highly effective and reproducible gene delivery. Other PEI or cationic polymers similar in structure thereto for transfecting cells are disclosed in U.S. Patent No. 6,013,240 and EP Patent No. 0770140.

The plasmids and the PEI are mixed before addition to the culture medium.

The N/P ratio is a measure of the ionic balance of the complexes. In the case of implementation of JetPEI® or PEIPro®, it refers to the number of nitrogen residues of JetPEI® per oligonucleotide phosphate. Preferably, the N/P ratio is under 10. In a specific embodiment, this ratio is of about 6. Optimizing this ratio allows for the optimal yield of lentiviral vector produced by the transfected cells associated with a limited toxicity.

The time during which the plasmids and PEI are in contact prior to the transfection step *per se* is also an important parameter, in order to properly complex the plasmid DNA to the PEI molecules. The present inventors have been able to demonstrate that contacting the plasmids with PEI during 5 to 30 minutes results in a mixture having very good transfection capacity. Preferably, the contact time will be of about 10 minutes to optimize the formation of the transfection complex.

The molar ratio between the different plasmids used for producing a lentivirus can also be adapted for optimizing the scale-up of this production. Thanks to the results provided herein, the person skilled in the art is able to adapt this parameter to the specific plasmids he uses for producing the lentivirus of interest. For example, the present inventors here show that a ratio of 1:1:2:1 (Env plasmid:Gag-Pol plasmid:Rev plasmid:TOI plasmid) leads to a more robust transfection and satisfying lentivirus production with respect to the lentiviruses shown in the examples. Of course, the person skilled in the art is able to adapt this ratio to the specific lentiviruses whose production is sought. The ratio can easily be adapted for each new situation (e.g. with respect to each specific plasmid vector used for the transfection) based on the teaching of the present invention (see the examples below) and common general knowledge in the field of recombinant lentivirus production. In particular, the molar ratio of the plasmids will be taken into account to optimize the quantity of each of these plasmids. This notion to use molar ratios rather than weight ratios is not obvious because in the field of the present invention, weight ratios are generally used for determining the quantity of each plasmid required for the production of a viral vector.

The person skilled in the art can adapt the transfection method to the particular cell culture implemented. In particular, the amount of total DNA (comprising in particular the four plasmids required for production of a recombinant lentivirus) can vary. However, in a specific embodiment of the invention, this amount will be of at least 1.5 µg/10⁶ cells. In a particular embodiment, the amount is of at least 2 µg/10⁶ cells, in particular of at least 2.5 µg/10⁶ cells. In a preferred embodiment, the amount of total DNA is of around 2 µg/10⁶ cells.

### Cell Culture

After transfection, for example after adding the mixture of DNA and PEI to the cell culture, this cell culture is allowed to grow for a time which can be comprised between 36 and 72 hours post-transfection, in particular after 48 hours.

In a particular embodiment, the medium used for culturing the cells is the same as the medium used for transfecting said cells. For example, in case of a transfection with a mixture of PEI and plasmid(s), the mixture may be done in F17 medium® and the cells may also be grown in said F17 medium® after transfection.

Culture may be carried out in a number of culture devices such as bioreactors adapted to the culture of cells in suspension. The bioreactor may be a single-use (disposable) or reusable bioreactor. The bioreactor may for example be selected from culture vessels or bags and tank reactors. Non-limiting representative bioreactors include a glass bioreactor (e.g. B-DCU® 2L-10L, Sartorius), a single-use bioreactor utilising rocking motion agitation such as wave bioreactor (e.g. Cultibag RM® 10L-25L, Sartorius), single use stirrer tank bioreactor (Cultibag STR® 50L, Sartorius), or stainless steel tank bioreactor. Growth is done under controlled condition (e.g. pH=7,2, pO2=50%, 37°C and a specific agitation according to the system, for culture of 293T cells as reported in the herein presented examples).

According to a particular aspect, the invention thus also relates to a cell culture device (i.e. a bioreactor) containing a volume of at least 5 L of a serum-free culture medium comprising mammalian cells transfected with at least one plasmid adapted for the production of a lentiviral vector, said cells being adapted to grow in suspension in said culture device. In a preferred embodiment, the cells are HEK 293T cells. In a particular embodiment, the culture device contains a volume of at least 10 L, at least 50, at least 100 L, at least 200 L or at least 1000 L of a serum-free culture medium as defined above. In other embodiments, the serum-free medium, transfection conditions, culture conditions and cells are as defined above.

The lentivirus may then be harvested (or collected), with one or more harvesting step. In a preferred embodiment, a single harvest of the lentiviruses present in the cell culture is done. This is a significant advancement of the invention over the prior art, where the available reports generally recommend several collections of the culture with numerous medium changes. Here, the preferred embodiment comprising a single harvest, without changing the culture medium from seeding into the bioreactor to the harvest, is a straightforward, cost-effective industrially compatible method. In a further particular embodiment, a single harvest is carried out 48 hours post-transfection. The lentivirus particles thus produced can then be harvested and purified according to methods also well known by the skilled artisan.

As mentioned above, 2 mM to 10 mM sodium butyrate, which is a known inducer of lentivirus production in adherent cell systems in the presence of serum, can be added to the culture medium. Unexpectedly in view of the conditions implemented herein (serum-free medium and suspension culture), the present inventors have shown that optimized production in suspension culture in the absence of serum can be obtained when sodium butyrate is added to the culture medium 24 hours post-transfection, and especially when it is used at a concentration of 5 mM.

### Further objects:

The invention also relates to a method for the large scale production of a recombinant lentiviral vector, comprising:
- transiently transfecting mammalian cells capable of suspension growth with a mixture of PEI and plasmids suitable for the production of a recombinant lentiviral vector;
- culturing the transfected cells in a serum free medium in batch culture in a volume of at least 5 L; and
- harvesting the produced recombinant lentiviral vector from the culture medium.
In a particular embodiment of this method, the plasmids include a plasmid encoding envelope proteins (Env plasmid), a plasmid encoding lentiviral Gag and Pol proteins (Gag-Pol plasmid), a plasmid encoding a lentiviral Rev protein (Rev plasmid) and a plasmid comprising a transgene of interest (TOI) expression cassette between a lentiviral 3'-LTR and a lentiviral 5'LTR. In a specific variant, transfection is carried out with a total DNA amount of at least 1.5 µg/10⁶ cells. Preferred cells are 293T cells adapted for suspension growth. In addition, in a particular embodiment, the cells are transfected with a mixture of polyethyleneimine (PEI) and DNA, wherein the PEI is a 20 - 25 kD linear PEI. In a specific variant, the PEI and the plasmids are mixed before transfection according to a N/P ratio of less than 10 (e.g. a ratio of around 6), wherein N/P refers to the number of nitrogen atoms in the PEI per oligonuclotide phosphate. Contact time between PEI and the plasmids before addition to the cell culture may be adapted, but is in particular comprised between 5 and 30 minutes, for example during around 10 minutes. Sodium butyrate may be added to the cell culture, for example 24 hours post transfection without changing the medium. Sodium butyrate final concentration in the culture may be comprised between 2 mM and 10 mM. Harvesting the cells may be carried out as a single harvest, in particular a single harvest between 48 hours and 72 hours post-transfection. The method of the invention may be carried out in a scale of at least 10 L, or more. This method may in particular relate to a method for high scale production of lentiviral vectors allowing harvesting at least 10⁷ infectious genomes/mL, preferably at least 3x10⁷ IG/mL.

The invention is further illustrated in the following non-limiting examples.

### BRIEF DESCRIPTION OF THE FIGURES

**Figure 1** is a graphical representation of the four plasmids used in the study presented in the examples.
**Figure 2** is a graph representing the test of different transfection conditions in 100 mL spinner flask with HEK293F cells and measurement of GFP positive cells by flow cytometry
**Figure 3** is a graph representing the test of different transfection conditions in 100 mL spinner flask with HEK293F cells and measurement of the amount of p24 HIV capsid antigen by p24 ELISA testing..
**Figure 4** is a graph representing the test of different transfection conditions in 100 mL spinner flask with HEK293T and measurement of GFP positive cells by flow cytometry.
**Figure 5** is a graph representing a test of different transfection conditions in 100 mL spinner flask with HEK293T cells and measurement of the amount of p24 HIV capsid antigen by p24 ELISA testing.
**Figure 6** is a graph representing the effect on production yield of two different SFM media for the generation of the transfection complex (F17 medium® and OptiProSFM®).
**Figure 7** is a graph showing the transfection at the optimal molar ratio (1:1:2:1) of plasmids on the production of two different products (different TOI) having different sizes. The assay was performed in spinner flasks at 100 mL under optimal transfection conditions.
**Figure 8** is a graph showing the impact of sodium butyrate addition strategy on productivity, measurement of the p24 concentation in supernatant
**Figure 9** is a graph showing the impact of sodium butyrate addition strategy on productivity, mesurement of the infectious genomes (IG) concentation in supernatant.
**Figure 10** is a graph showing the impact of sodium butyrate addition strategy on productivity, measurement of the ratio physical particles / infectious particles (PP/IP) in supernatant.
**Figure 11** is a graph representing the average of 6 productions of HIV-VSVG-WASp in spinner flask at 100 mL with addition of sodium butyrate 24 hpt at a 5 mM final concentration in the culture.
**Figure 12** is a graph showing the comparison between suspension protocol at 100 mL with HEK293T grown in suspension in a serum-free medium and the standard in 10 stacks Cell Factories for production of HIV-VSVG-WASP lentiviral vector, IG results and PP/IP ratio in supernatant at 48 hpt.
**Figure 13** is a graph representing the evaluation of the suspension process of the invention at different scales (100 mL to 50 L) in different cell culture devices (spinner, wave and stirrer tank) and comparison with conventional adherent cells process using serum.

### EXAMPLES

The aim of this study was to produce a lentiviral vector at a scale compatible with industrial applications, in a bioreactor in suspension in a serum free media. Advantageously, the process has been developed up to 50 L and the production is readily adaptable to at least 100 L, 200L bioreactor scale, or even at least 1000 L.

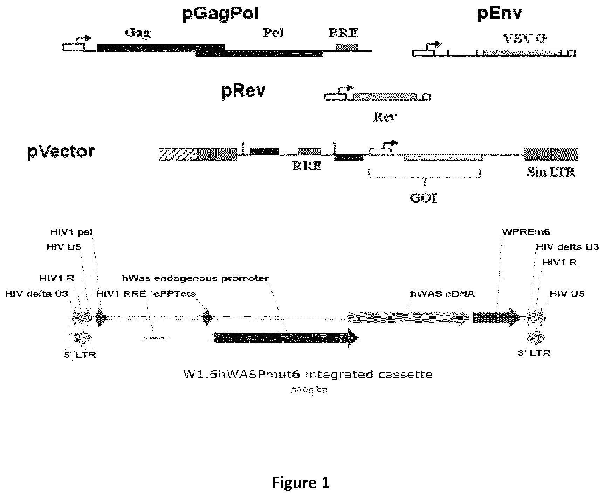

For recombinant lentivirus production we used 4 plasmids (see strategy in figure 1).

### Materials and Methods

### Cell Culture:

All vector production and cell culture were done with an anchorage dependent HEK293T working cell bank (WCB), initially growing in the presence of fetal bovine serum which was adapted for suspension growth in serum free media and a new working cell bank was established. Cells were cultured in modified F17 medium® supplemented with Pluronic® F68 (Invitrogen), GIBCO® Anti-Clumping Agent (Invitrogen) and 4 mM GlutaMAX™ (Invitrogen). For the process development described, different culture containers were used under controlled conditions:
- spinner flask (Techne, UK) for the 100mL scale under controlled conditions (37°C, 120 rpm)
- for larger scale: glass bioreactor (B-DCU® 2L-10L, Sartorius), wave bioreactor (Cultibag RM® 10L-25L, Sartorius), single use stirrer tank bioreactor (Cultibag STR® 50L, Sartorius) under controlled condition (pH=7,2, pO2=50%, 37°C and a specific agitation according to the system)

### Vectors and plasmids:

The W1.6-huWASP-WPRE vector described in (Zanta-Boussif et al., 2009, Gene Ther.; 16(5):605-19), was produced by transient transfection of 293T cells using 4 plasmids consisting pCCL W1.6-huWASP-WPREmut6-K transfer plasmids combined with the GagPol, VSV-G, Rev plasmids respectively coding for HIV-1 *gag-pol*, *rev* genes and for the unrelated vesicular stomatitis virus G glycoprotein. All plasmids contain the kanamycin resistance gene. Further details are provided in Merten et al., cited *supra*.

The HIV-VSVG-GFP vector was produced using the same reagent except for the transgene plasmid which is pRRLSINcPPT-PGK-eGFP-WPRE.

### Sodium butyrate:

Sodium butyrate is commercially available (sodium butyrate ≥98.5% (GC) | Sigma-Aldrich). A stock solution is prepared at 500 mM in customized F17 medium® and 0,22 filtered.

### Medium:

F17 medium® (Invitrogen) is customized by supplemention with Pluronic® F68 at 0,08%, GIBCO® Anti-Clumping Agent at 0.01 %, and GlutaMAX™ at 4 mM final concentration.

### Lentiviral vector production:

Suspension culture vessels or bags were seeded at 0,2x10⁶ cells/mL. Transfection was performed 72h after seeding. Cell density was between 0.8 and 1.3x10⁶ cells/mL at the time of transfection.

### Example for WASp production (best mode):

The four plasmids used in this study are represented in figure 1. Different amounts of total DNA have been tested. Different concentrations have been tested but in the most optimal conditions total DNA was used at an amount of around 2 µg/10⁶ cells. The transfection reagent used was JetPEI® (Polyplus product) with an N/P ratio of about 6. DNA and JetPEI® were respectively diluted in culture media before being gently mixed for approximately 10 min. This mixing led to the formation of a transfection complex, which was directly added to the cell culture. Twenty four hours after transfection, sodium butyrate was added for a final concentration of approximately 5mM. Conditioned media containing the lentiviral vector particles were harvested 72h after transfection for analytical purposes.

### Titration:

Physical particles produced were quantified by measuring the amount of p24 (HIV capsid protein) using a specific ELISA kit. Infectious particles were titrated after infection of a cell line susceptible to lentiviral vector infection using qPCR (TaqMan) as previously described in Merten et al. (*supra*).

### Results

### A-Description of adaptation of HEK293T cells to suspension culture in chemically defined media in the absence of serum

### -Source of the HEK293T cell line:

Cells came from a vial of an adherent, GMP master (working) 293T cell bank cultured in DMEM at 10% FBS.

### -Adaptation to suspension in the serum free media:

Cells were thawed in a T75 tissue culture flask (DMEM + 10 % FBS). After 2 passages and amplification in a T175 tissue culture flask, we performed a complete media change on adherent cells replacing DMEM by modified F17 medium® (serum free media). 48h after media change, all cells were detached from the support and viability was still around 90%. Cells were continuously cultivated in F17 in T175 tissue culture flask. After 3 passages in F17 and amplification in T225 tissue culture flask, cells were seeded in spinner flask at 50ml in suspension condition (using single use spinner flask, Corning). A cell bank of 54 vials (50x10⁶ cells/vial) of 293T cells in suspension was generated at passage 8 (P8).

### -Generation of cell bank.

The formulation for cryoconservation is: 80% F17, 10% DMSO and 10% methylcellulose 1%.

### B-Production of lentiviral vector expressing the green fluorescent protein in classical HEK293F vs. HEK293T

One of the aims of our work was to establish a process for manufacturing lentiviral vectors in suspension culture in the absence of serum for industrial applications.
Initially, experiments were performed with HEK293F cell line, a commercially available cell line adapted for suspension culture in the absence of serum.
To generate the HIV-GFP lentiviral vectors by the 4-plasmid transfection system described in (Zanta-Boussif et al., 2009, Gene Ther.; 16(5):605-19), HEK293F cells were seeded in 100 mL Spinner flasks at 1E+06 cells/mL. Different transfection conditions were tested at a scale of 100 mL: Variable parameters were: the amount of total DNA used per 1x10⁶ cells, as well as the molar ratio between the 4 plasmids/ 1E+06cells. Although variations are possible in these parameters the contact time for the complex formation (10 min) with transfection reagent (JetPEI®) and the ratio DNA/PEI (N/P=6) were fixed as the optimal conditions for lentivirus production.
The DNA/PEI complex was generated in 10mL of OptiProSFM® (Invitrogen), a chemically defined media. After 10 minutes of contact, the DNA/PEI complex mix was added directly into the culture.
To assess efficiency of transfection, cell cultures were analyzed by flow cytometry to measure green fluorescent protein expression.
In addition cell culture supernatants were subjected to p24 ELISA testing to measure the concentration of the HIV p24 capsid antigen which is indicative of the presence of lentiviral particles.

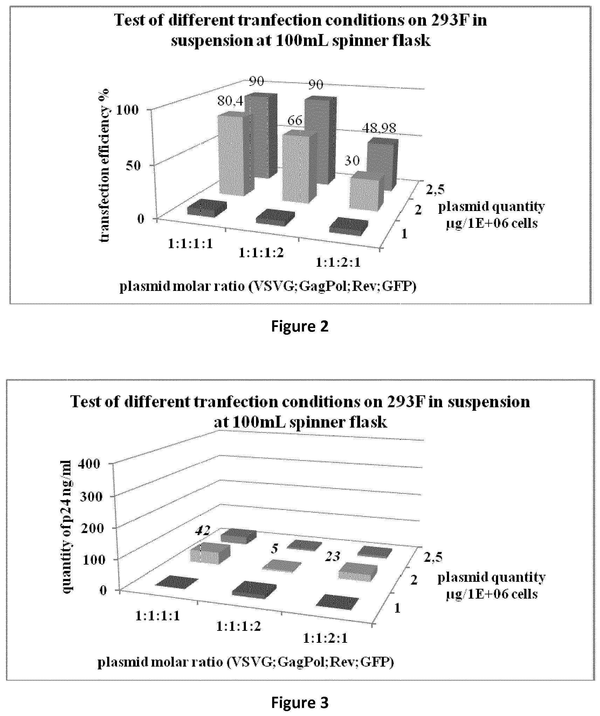

The results are presented in Figure 2 and 3.

### Transfection efficiency 48h post transfection:

Results show that even if HEK293F can be efficiently transfected in certain conditions of DNA concentration and ratio (2.5µg, 1:1:1:1 and 1:1:1:2, respectively), very little amounts (<50ng/mL) of p24 antigen can be detected. An amount of p24 above 150 ng/mL is indicative of an efficient lentiviral production whereas a lower value is essentially due to free p24. We can correlate the amount of p24 with the amount of physical particles using an ELISA Kit (Alliance HIV-1 P24 ANTIGEN ELISA Kit (480 Test), PerkinElmer) which gives this information: 1 ng p24 = 1,2x10⁷ PP

### C-Production of HIV-GFP from HEK293T

The production of the lentiviral vector HIV-GFP was performed in similar conditions using HEK293T cells. The efficiency of transfection and the concentration of p24 antigen in the cell culture supernatants were determined at 48h post transfection.

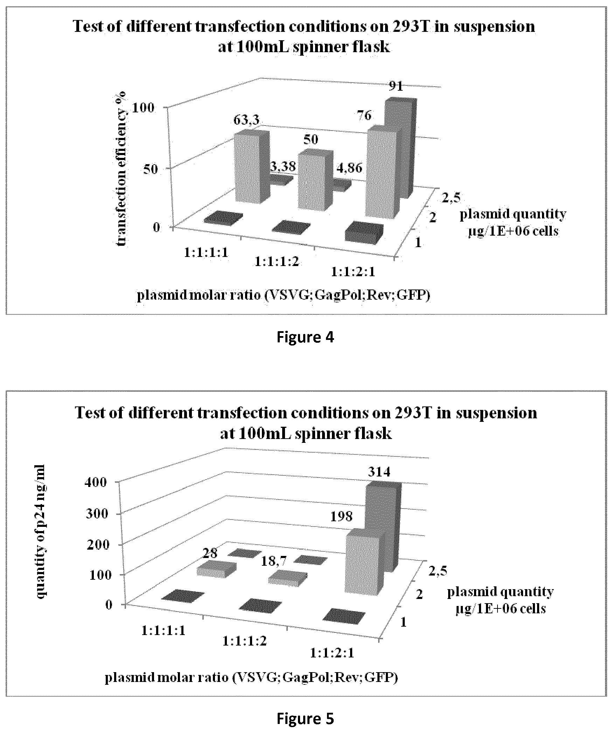

The results are presented in Figures 4 and 5.

Results show that at a similar efficiency of transfection (∼90% at 2 and 2.5µg DNA, ratio 1:1:2:1), HEK293T cells are more efficient than HEK293F at generating p24 antigen and therefore HIV lentiviral vector particles (198 ng/mL and 314ng/mL).
In conclusion, these experiments allowed us to determine efficient conditions to generate lentiviral vectors in HEK293T cells at small scale. Those conditions were further investigated to evaluate the feasibility to manufacture lentiviral vectors in suspension in the absence of serum at a scale allowing industrial applications.

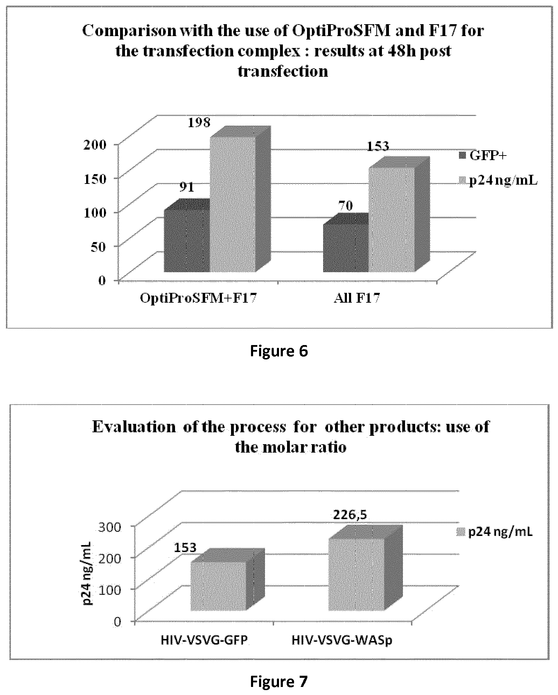

D-Optimization of the lentiviral vector production process in HEK293T cells in suspension in the absence of fetal bovine serum
D1-Elimination of the OptiProSFM® medium for PEI/DNA complex generation.
To simplify the process, we investigated the possibility to generate the PEI/DNA complex directly in the F17 medium® so as to avoid the use of a different media (OptiProSFM®.).
Lentiviral vector production was performed at 100 mL scale in a spinner flask using the transfection conditions determined in previous experiments, i.e., use of HEK293T cells, plasmid molar ratio of 1:1:2:1 and 2,5µg total DNA /1x10⁶ cells. Cells and supernatants were harvested for testing and results are presented in Figure 6.

Results show that there is no major difference in p24 concentration if generated from PEI/DNA complexed in the Optipro media vs. F17. Using F17 media only throughout the process, rather than using two different types of media, constitutes a major improvement towards adapting the process to industrial scale.

D2-Importance of using plasmid molar ratio instead of plasmid (DNA) mass ratio in the production system - Flexibility of the process thanks to the use of a molar ratio:
The lentiviral vector system of production used in the present experiments involves 4 plasmids. Three of those (Gag-Pol plasmid, VSV-G plasmid and Rev plasmid) are common to all lentiviral vectors as they encode trans acting functions necessary for the formation of the lentiviral particles themselves, i.e. the structural elements (vector capsid, VSV-G envelope), enzymatic proteins (reverse transcriptase, integrase), and regulatory factor of expression (Rev protein). The only factor that varies is the plasmid encoding the vector genome. Because the transgene expression cassette encoded by the vector genome plasmid can come in different sizes (different promoters, cDNAs), the final amount of plasmid necessary for the generation of functional particles can vary from vector to vector, and with different expression cassettes.

Therefore, given that the molar ratio 1:1:2:1 (Env plasmid:Gag-Pol plasmid:Rev plasmid:TOI plasmid) gave the best results we wanted to verify that by keeping the molar ratio intact, we could reproducibly maintain lentiviral production yields even if the size of the plasmid varied. Thanks to this molar ratio, which keeps the number of each plasmid molecules intact independently of their size in base pair (and therefore their weight), we can guarantee the optimal transfection conditions regardless of the product.

Figure 7 shows an example where we compared a lentiviral vector encoding the GFP protein cDNA (total size of the plasmid = 7388 bp) with a lentiviral vector encoding the Wiskott-Aldrich protein (WASp) cDNA (total size of the plasmid = 9780 bp).

The results show that by keeping the ratio of plasmid equal in terms of number of molecules (which conversely affects the amount of individual plasmid used), the yields of lentiviral vectors remain intact. These results suggest that the method of production of lentiviral vectors in HEK293T cells, in suspension in the absence of serum with a total of 2.5µg/1E+06 cells at a molar ratio of 1:1:2:1 can be used for different vectors independently of size. Of course, although optimal, these conditions may be varied starting from these values so that a person skilled in the art can adapt the parameters to the particular cells and plasmids used for production of the desired lentiviral vector

### D3-Improvement of productivity: use of sodium butyrate

Sodium butyrate has been reported to enhance the production of lentiviral vectors in an adherent cell system.(*Gasmi et al* Mar. 1999). We wanted to determine whether sodium butyrate would be useful in such suspension cultures. However, if that was the case, the use of sodium butyrate should not make the process more cumbersome, the priority being to keep the process applicable to industrial applications. Therefore we decided to test whether the addition of sodium butyrate post transfection without media change could impact positively lentiviral vector yields in the conditions previously established (HEK293T, suspension culture, absence of serum, 2.5µg/mL plasmid at a ratio of 1:1:2:1).
Experiments were performed at the 100 mL scale, in spinner flasks. Sodium butyrate was prepared in customized F17 medium® and added post transfection at a final concentration of 5mM. The effect on lentiviral vector production was assessed by measurement of p24 antigen by ELISA and by mesuring the concentration of infectious particles using qPCR (TaQman), Measuring both parameters allows for the calculation of the PP/IP ratio (total number of particles to infectious particles) which is an indicator of the quality of a lentiviral vector preparation. The quality of the production is considered as acceptable when the ratio PP/IP is between 100-250 (results commonly observed for GMP production at GENETHON)

Initially we tested differents strategies for sodium butyrate added in a 100 mL spinner flask. One strategy was to add it 6h post transfection directly in the culture. An another strategy was to perform a complete media change 24h post transfection and add the sodium butyrate in the fresh media used to resuspend cells. Finally, the strategy giving the best results was to add directly the sodium butyrate in the culture 24h post transfection without media change. Note : during the media change cells were centrifuged 5min at 500g before resuspending them in fresh F17.

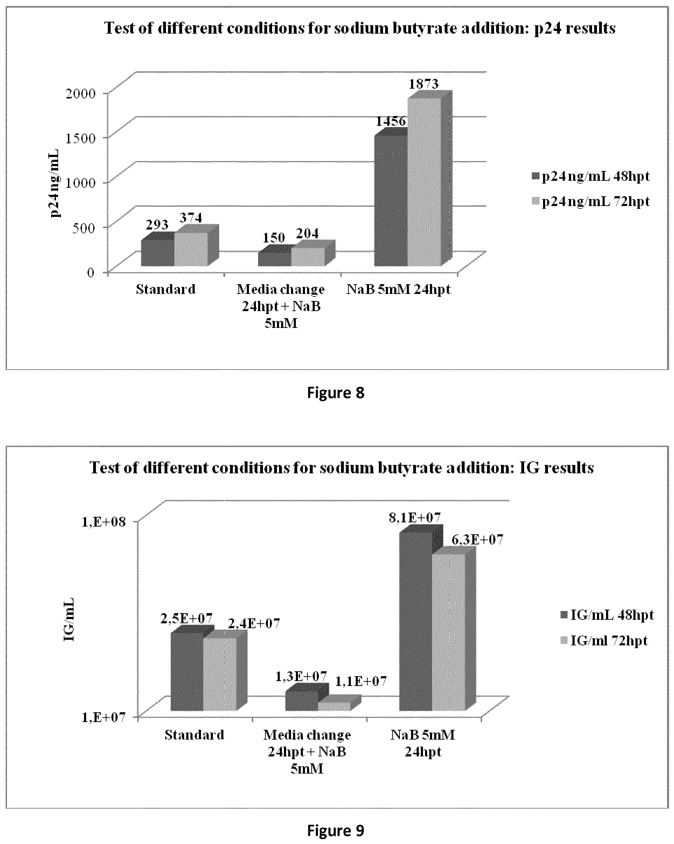

We performed an experiment in parallel with three spinners to confirm previous experiments, results are in figures 8, 9 and 10.

Results show that adding sodium butyrate at a final concentration of 5mM, 24h post transfection increases vector productivity between 3-4 fold concerning infectious particles and that there is also an increase of the amount of p24 produced.

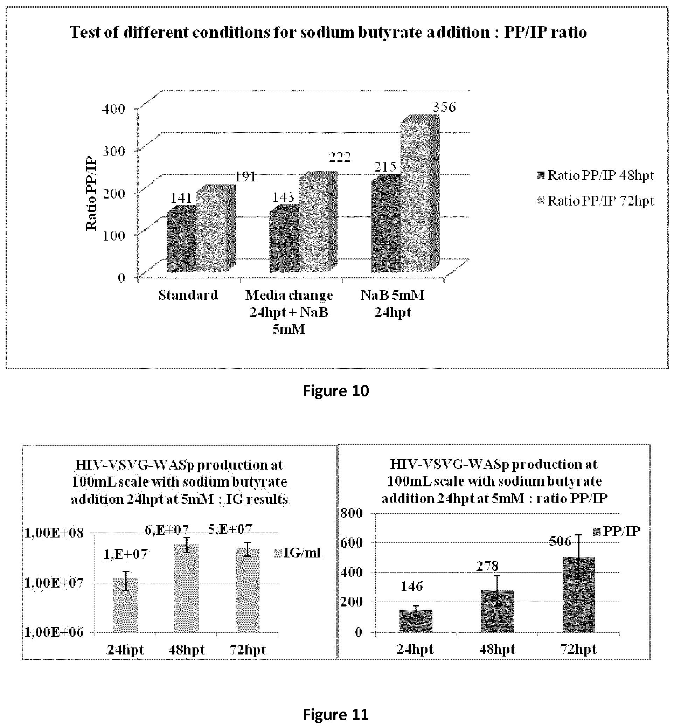

Figure 10 presents the ratio PP/IP that we had for this experiment.

This graph shows that sodium butyrate allows not only an increase of productivity but also keeps an acceptable quality of the production by giving a PP/IP ratio in the acceptable range (100-250).

The robusteness of this strategy was assessed by doing 6 spinners with the same protocol, results are represented in figure 11.

These experiments confirm that the better harvest time is 48 hpt according to the IG concentration and the quality of the production regarding the PP/IP ratio at 48 hpt.

### E-Scale up.

E1- Demonstration that the lentiviral vector production method in suspension-grown cells in the absence of serum gives similar results as the conventional lentiviral vector production system in adherent cells in the presence of serum.

Commonly, large scale productions of lentiviral vectors for research or clinical purposes are performed using transfection of HEK293 adherent cells in the presence of serum. For reason of vector titers, HEK293T are the most commonly used cells. The production protocols are essentially based on the use of 2 stacks or 10 stacks cell factories or equivalent multitray systems. See Schweizer and Merten, 2010 Current Gene Therapy 10(6), 474-486, most particularly part 2.3 ("large scale process, Including Transient Transfection")

This adherent-cell based protocol has been compared to the optimal method defined above in which HEK293T cells were grown in suspension in the absence of serum, and transfected with 2.5µg DNA/1x10⁶ cells with a plasmid molar ratio of 1:1:2:1 with sodium butyrate added at 5 mM 24 hpt without media change.

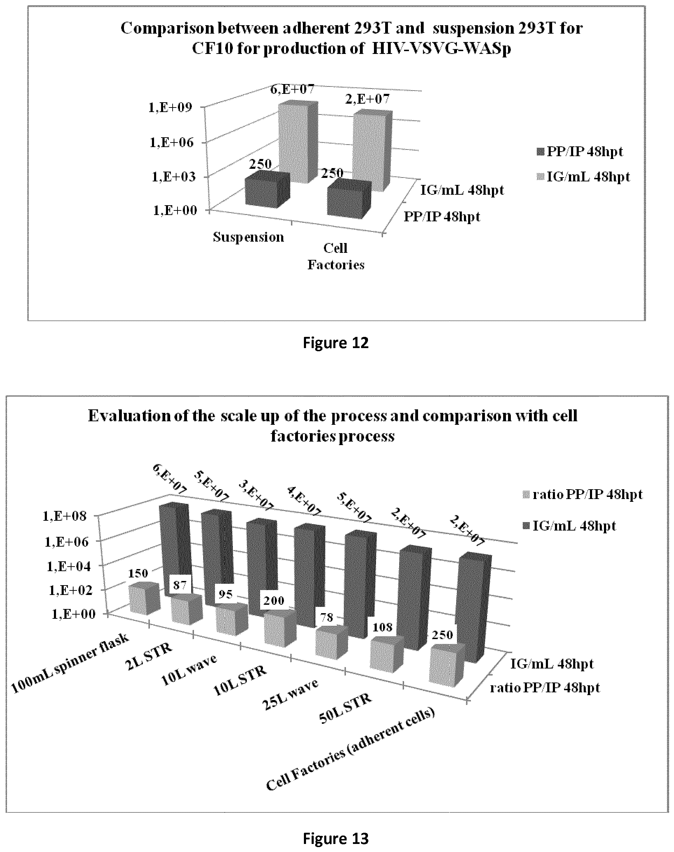

Figure 12 shows a comparison between suspension protocol at 100 mL with HEK293T and the standard in 10 stacks cell factories for production of HIV-VSVG-WAS lentiviral vector.

Results demonstrate that the suspension system generates lentiviral vectors in similar yields and quality as the adherent cell system.

E2-Demonstration that the lentiviral vector production system in suspension in the absence of serum according to the invention can be scaled up and applied to industrial applications.

The scalability of the optimal process of lentiviral production described above (HEK293T cells in suspension in the absence of serum with 2.5µg/mL DNA/1e6 cells at a plasmid ratio of 1:1:2:1 with sodium butyrate) was evaluated over various volumes of culture in terms of yields of particles (p24 ELISA) and infectious particles (qPCR ,TaqMan) in the case of a lentiviral vector HIV-VSVG-WASp. Ratios of PP/IP as described before were calculated for each scale and plotted in the histogram presented in figure 13 in comparison with results obtained with the conventional production method in adherent cells in the presence of serum.

Results show that vector productivity (number of infectius genomes, IG) and quality (PP/IP) of the novel system of lentiviral vector production is maintained over a wide range of culture volumes and that they favorably compare with those obtained with the conventional method of production implementing adherent cells grown in a serum-containing medium (same quality and productivity for all scale and competitive with the Cell factories process).

These results show that the novel process of lentiviral vector production in suspension combines efficiency with practicality and can therefore be used in industrial scale applications of lentiviral vectors.

## Claims

1. A method for the production of a recombinant lentiviral vector, comprising:
- culturing, in suspension in a serum-free medium, mammalian cells transfected with at least one plasmid adapted for the production of a lentiviral vector, the culture being carried out in a volume of at least 5 L;
- harvesting the produced recombinant lentiviral vector from the culture medium.

2. The method according to claim 1, wherein the mammalian cells are HEK293T cells.

3. The method according to claim 1 or 2, wherein the harvesting step consists of a single lentivirus harvest.

4. The method according to claim 3, wherein the transfection is a transient transfection and the single harvest is implemented between 48 and 72 hours post-transfection.

5. The method according to any one of claims 1 to 3, comprising a transfection step wherein the cells are transfected with a mixture of polyethylenimine (PEI), such as a 20 - 25 kD linear PEI, and plasmids.

6. The method according to claim 5, wherein transfection is carried out with a total DNA amount of at least 1.5 µg/10⁶ cells.

7. The method according to claim 5 or 6, wherein the PEI and the plasmids are mixed before transfection according to a N/P ratio of less than 10, such as of around 6, wherein N/P refers to the number of nitrogen atoms in the PEI per oligonuclotide phosphate.

8. The method according to any one of claims 5 to 7, wherein the contact time between PEI and the plasmids before addition to the cell culture is comprised between 5 and 30 minutes, the contact time being in particular of around 10 minutes.

9. The method according to any one of claims 1 to 10, wherein sodium butyrate is added to the cell culture 24 hours after transfection of the cells without changing the medium, preferably wherein sodium butyrate is added to the cell culture at a final concentration in the culture comprised between 2 mM and 12 mM, in particular between 2 mM and 10 mM, more particularly at a final concentration of 5 mM.

10. The method according to any one of claims 1 to 11, wherein the cells are transfected with four plasmids including a plasmid encoding the envelope proteins (Env plasmid), a plasmid encoding the lentiviral GagPol proteins (Gag-Pol plasmid), a plasmid encoding the lentiviral Rev protein (Rev plasmid) and a plasmid comprising a transgene of interest (TOI) between a lentiviral 3'-LTR and a lentiviral 5'LTR (TOI plasmid).

11. The method according to any one of claims 1 to 10, wherein
- the culture is implemented in a volume of at least 50 L; and/or
- wherein at least 10⁷ infectious genomes /mL are produced.

12. The method according to any one of claims 1 to 11, wherein:
- the cells are 293T cells;
- transfection of the cells is carried out with a mixture of PEI and the required plasmid(s);
- sodium butyrate is added 24 hours post-transfection without changing the medium of the culture; and
- a single harvest of produced lentiviral vectors is carried-out.

13. A cell culture device, wherein said culture device contains a volume of at least 5 L of a serum-free culture medium comprising mammalian cells, such as HEK 293T cells, transfected with at least one plasmid adapted for the production of a lentiviral vector, said cells growing in suspension in said culture device.

14. A method for optimizing the production of a lentiviral vector by a mammalian cell grown in suspension in a serum-free medium, transfected with plasmids required for said production, comprising adding sodium butyrate 24 hours post-transfection to a cell culture without changing the medium of the culture.

15. The method according to claim 14, wherein sodium butyrate is added at a final concentration of 5 mM.
